# EUROPEAN PATENT APPLICATION

(11) **EP 1 721 628 A1**
(43) Date of publication of application: **15.11.2006**
(21) Application number: 06009141.0
(22) Date of filing: 03.05.2006
(51) Int. Cl.: A61M 15/00, A61M 15/08, B05B 11/06

(54) **Powder medicine administering device**

(30) Priority: 13.05.2005 JP 2005141496
(71) Applicant: HITACHI, LTD., Chiyoda-ku Tokyo 100-8280 (JP); DOTT LIMITED COMPANY, Yokohama-shi, Kanagawa 224-0051 (JP)
(72) Inventor: Ishizeki, Kazunori, Isesaki-shi Gunma 372-0023 (JP); Ohki, Hisatomo, Isesaki-shi Gunma 372-0023 (JP); Nakamura, Shigemi, Isesaki-shi Gunma 372-0023 (JP); Yanagawa, Akira, Tsuzuki-ku,Yokohama-shi Kanagawa224-0024 (JP)
(74) Representative: Weber, Joachim

(57) **Abstract**

A powder medicine administering device (1) includes a main body (2) formed with a powder medicine discharge passage (9,10) for discharging a powder medicine (16), and a nozzle member (4;21) removably attached to the main body, and formed with a nozzle passage (14) connected with the powder medicine discharge passage of the main body, and arranged to receive the powder medicine in a closed state. The nozzle member is arranged to be changed from the closed state to an opened state when the nozzle member is attached to the main body, to discharge the powder medicine received in the nozzle passage.

## Description

### BACKGROUND OF THE INVENTION

The present invention relates to a powder medicine administering device to administer a powder medicine.

Japanese Patent Application Publication No. S59 (1984)-34267 shows a powder medicine administering device including a needle for forming a hole in a capsule made from gelatin, and filled with a powder medicine. The powder medicine administering device is arranged to discharge the powder medicine from the hole formed by using the needle.

### SUMMARY OF THE INVENTION

The above-mentioned powder medicine administering device can vary medicines for administration and dose of the medicine readily.

However, in the above-mentioned powder medicine administering device, when the capsule is deteriorated, it is difficult to make a predetermined hole in the capsule by using the needle. Consequently, the powder medicine may not be discharged, and discharge quantity of the powder medicine may be decreased.

It is an object of the present invention to provide a powder medicine administering device devised to reduce and eliminate defects produced by using a capsule, without using the capsule filled with powder medicine.

According to one aspect of the present invention, a powder medicine administering device comprises a main body formed with a powder medicine discharge passage for discharging a powder medicine; and a nozzle member removably attached to the main body, and formed with a nozzle passage connected with the powder medicine discharge passage of the main body, and arranged to receive the powder medicine in a closed state, the nozzle member being arranged to be changed from the closed state to an opened state when the nozzle member is attached to the main body, to discharge the powder medicine received in the nozzle passage.

According to another aspect of the invention, a powder medicine administering device comprises: a main body; and a nozzle member removably attached to the main body, and formed with a nozzle passage for discharging a powder medicine, the nozzle passage being arranged to receive the powder medicine.

### BRIEF DESCRIPTION OF THE DRAWINGS

FIG. 1 is a longitudinal sectional view showing a powder medicine administering device according to a first embodiment of the present invention.

FIG. 2 is a longitudinal sectional view showing a powder medicine receiving container for the powder medicine administering device of FIG. 1.

FIG. 3 is a perspective view showing a receiving case for receiving the powder medicine administering device of FIG. 1.

FIG. 4 is a sectional view showing a powder medicine receiving container according to a second embodiment of the present invention.

FIG. 5 is a sectional view showing a powder medicine receiving container according to a third embodiment of the present invention.

FIG. 6A is a longitudinal sectional view showing a powder medicine receiving container according to a fourth embodiment of the present invention. FIG. 6B is a plan view showing a bottom portion of the powder medicine receiving container of FIG. 6A.

FIG. 7 is a perspective view showing an upper portion of a main body for supporting the powder medicine receiving container of FIG. 6A.

FIG. 8A is a side view illustrating mounting operation of the powder medicine receiving container of FIG. 6A before the powder medicine receiving container is mounted to the main body. FIG. 8B is a side view showing the powder medicine receiving container of FIG. 6A and the main body after the powder medicine receiving container is mounted to the main body.

FIG. 9A is a longitudinal sectional view showing a powder medicine receiving container according to a fifth embodiment of the invention. FIG. 9B is a plan view showing a bottom portion of the powder medicine receiving container of FIG. 9A.

FIG. 10 is a perspective view showing an upper portion of a main body to which the powder medicine receiving container of FIG. 9A is attached.

### DETAILED DESCRIPTION OF THE INVENTION

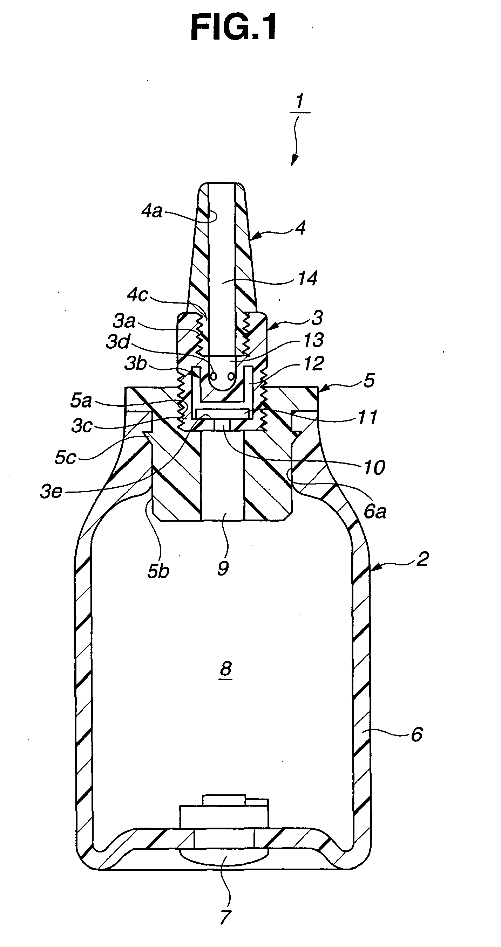

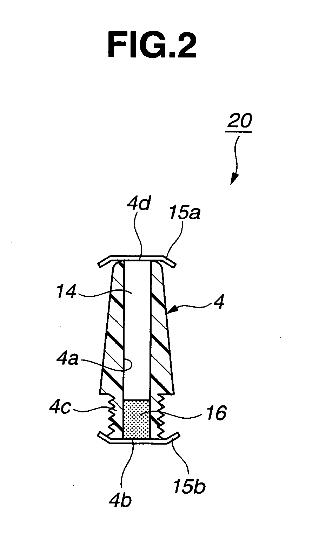

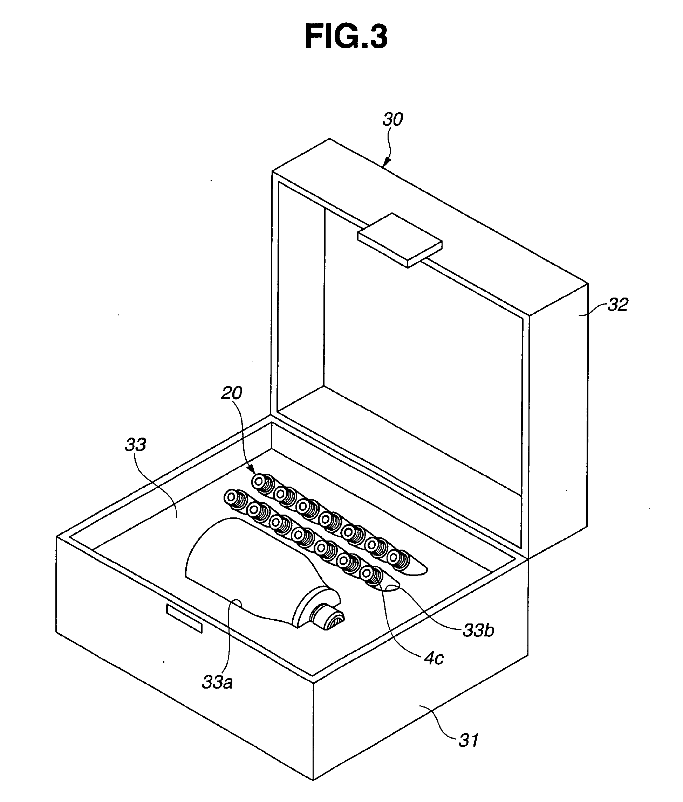

(First embodiment) FIG. 1 shows a longitudinal sectional view showing a powder medicine administering device according to the first embodiment of the present invention. FIG. 2 shows a longitudinal sectional view showing a powder medicine receiving container of the powder medicine administering device of FIG. 1. FIG. 3 shows a perspective view showing a receiving case for receiving the powder medicine administering device and the powder medicine receiving containers. The powder medicine administering device of FIG. 1 is a take-along powder medicine administering device for administering powder medicine into nasal cavities.

A powder medicine administering device 1 includes a main body 2 and a nozzle member 4. Main body 2 is formed with medicine discharge passages (9, 10 and so on). Nozzle member 4 is formed with a nozzle passage 14, and connected with the medicine discharge passages. Main body 2 includes a pump member 6, a base member 5, a stirred flow forming member 3, and an air introduction member 7. Pump member 6 serves as an air supply mechanism or section arranged to supply the air to the medicine discharge passages for discharging the powder medicine. Base member 5 is formed with air passage or medicine discharge passage 9 connected with a pump chamber 8 within pump member 6. Stirred flow forming member 3 is attached to base member 5, and provided with a stirred flow forming mechanism or section. Air introduction member 7 introduces the air into pump member 6. Powder medicine administering device 1 according to the first embodiment is shaped like a body of revolution having axis extending in an up-down direction of FIG. 1.

Nozzle member 4 is detachably or removably attached to main body 2. Nozzle member 4 includes an outlet opening or discharge opening 4d, and an external thread portion 4c located at a position opposite to outlet opening 4d. Stirred flow forming member 3 includes an internal thread portion 3a into which external thread portion 4c of nozzle member 4 is screwed. Nozzle member 4 is mounted to stirred flow forming member 3 by the screw connection between external thread portion 4c and internal thread portion 3a. Accordingly, nozzle member 4 can be attached to or detached from main body 2 readily by the screw connection between external thread portion 4c and internal thread portion 3a.

Nozzle member 4 includes a through hole 4a serving as a nozzle passage 14. Stirred flow forming member 3 includes a stirred flow forming chamber 13. Nozzle passage 14 is connected with stirred flow forming chamber 13 when nozzle member 4 is mounted to stirred flow forming member 3.

Stirred flow forming member 3 is attached to base member 5. In this example, stirred flow forming member 3 includes an external thread portion 3c located at a position opposite to internal thread portion 3a. Base member 5 includes an internal thread portion 5a into which external thread portion 3c of stirred flow forming member 3 is screwed. Stirred flow forming member 3 is attached to base member 5 by the screw connection between external thread portion 3c and internal thread portion 5a. Accordingly, stirred flow forming member 3 can be attached to or detached from main body 5 readily by the screw connection between external thread portion 3c and internal thread portion 5a. Therefore, it is advantageous to change stirred flow forming member 3 appropriately in accordance with kind of the medicine, and to produce the stirred flow suitable for the medicine.

Stirred flow forming member 3 is provided with the stirred flow forming mechanism. Stirred flow forming member 3 includes air passage or medicine discharge passage 10 and an intermediate chamber 12 being in the form of a body of revolution with a substantially U-shaped section. Air passage 10 is formed on a base member 5's side (a lower side in FIG. 1) of stirred flow forming member 3 in FIG. 1, and connected with air passage 9 of base member 5. Intermediate chamber 12 is connected with a downstream end portion of air passage 10 (an upper end portion of air passage 10 in FIG. 1). A sheet-shaped check valve or nonreturn valve 11 is provided on a bottom wall surface 3e of intermediate chamber 12, so as to prevent backflow of the air and the medicine. Check valve 11 is arranged to open or close an inlet portion of intermediate portion 12 (an outlet portion of air passage 10). Stirred flow forming member 3 includes a wall portion 3b being in the form of a cylindrical shape with a bottom, and including a circumferential wall and a bottom wall. Intermediate chamber 12 is separated from stirred flow forming chamber 13 by wall portion 3b of stirred flow forming member 3. A plurality of through holes 3d (four through holes in the first embodiment) are formed in the circumferential wall of wall portion 3b. Each through hole 3d is inscribed in an inner surface of the circumferential wall of wall portion 3b, as viewed from above. Through holes 3d connect intermediate chamber 12 and stirred flow forming chamber 13. Stirred flow forming member 13 receives the powder medicine provided in nozzle passage 14, and serves as a medicine receiving chamber for restricting the reverse flow in the upstream direction.

By this arrangement, swirl flow (vortex flow) is formed as the stirred flow in stirred flow forming chamber 13. The air is introduced through pump chamber 8, air passages 9 and 10, check valve 11, intermediate chamber 12, and through holes 3d into stirred flow forming chamber 13. Each of through holes 3d is formed along a tangent line of the inner surface of the circumferential wall of stirred flow forming chamber 13. The swirl flow is formed in stirred flow forming chamber 13 along the inner surface of the circumferential wall of stirred flow forming chamber 13. This stirred flow flows downstream to nozzle member 4, and curls up the powder medicine. Accordingly, it is possible to improve discharge efficiency of the introduced powder medicine, and to expel to further diffuse the medicine.

In this arrangement, the powder medicine is introduced to nozzle passage 14 as described later. The powder medicine is introduced downwards from nozzle passage 14 to stirred flow forming chamber 13. Each through hole 3d serving as the air passage is located at a position upstream of nozzle passage 14. Each through hole 3d is directed in a direction perpendicular to the introducing direction (up-down direction) of the powder medicine, and located at a position separated from the bottom of cylindrical wall portion 3b. Moreover, wall portion 3b is so arranged as to restrict the backflow of the powder medicine to intermediate chamber 12. Check valve 11 is so arranged as to restrict the back flow in a direction of pump chamber 8. By this arrangement, it is possible to restrict the powder medicine from remaining.

A claw portion 5c is formed on a circumferential outer surface 5b of base member 5 so as to protrude outwards. A through hole 6a of an upper portion of pump member 6 is retained by claw portion 5c to abut on outer surface 5b of base member 5. When the user presses pump member 6, pump chamber 8 is contracted. Then, the compressed air in pump chamber 8 is transferred through air passages 9 and 10, stirred flow forming chamber 13, and so on, to nozzle passage 14. In the device according to the first embodiment, air passages 9 and 10, intermediate chamber 12, through holes 3d, and stirred flow forming chamber 13 are formed in main body 2, are the air passages to discharge the introduced powder medicine outwards, and correspond to the medicine discharge passages according to the invention.

As shown in FIG. 2, a powder medicine receiving container 20 includes sheet-shaped seal members 15a and 15b, and nozzle member 4. Powder medicine 16 is received in nozzle passage 14 of nozzle member 4. Seal members 15a and 15b close or seal nozzle passage 14 to restrict the leakage of powder medicine 16. For example, seal member 15a (for example, a film) is attached (or stuck) to an end surface of nozzle member 4 in which outlet opening or discharge opening 4d is formed. Seal member 15b (for example, a film) is attached (or stuck) to an end surface of nozzle member 4 in which an inlet opening or introduction opening 4b is formed. Seal members 15a and 15b are attached to be readily detached, and so as not to remain glue after the detachment.

Hereinafter, the use of powder medicine receiving container 20 and powder medicine administering device 1 is illustrated. First, powder medicine receiving container 20 is disposed to position inlet opening 4b (external thread portion 4c) above, and to position outlet opening 4d below (that is, in a posture to reverse the upper and lower sides in FIG. 2). In this posture, seal member 15b on upper side (inlet opening 4b's side) of powder medicine receiving container 20 is detached or removed. Main body 2 from which nozzle member 4 is detached is disposed to position stirred flow forming member 3 below, and to position pump member 6 above. Then, main body 2 is rotated, and external thread portion 4c of nozzle member 4 is screwed into internal thread portion 3a of stirred flow forming member 3, so that nozzle member 4 is attached to main body 2.

Second, main body 2 to which nozzle member 4 is attached, that is, assembled powder medicine administering device 1 is turned upside down to position nozzle member 4 above, and to position pump member 6 below. At this time, the powder medicine in nozzle passage 14 of nozzle member 4 drops into stirred flow forming chamber 13 serving as the medicine receiving chamber.

Third, seal member 15a located on outlet opening 4d's side of nozzle member 4 is detached, and nozzle member 4 is inserted into nasal cavity. Then, pump member 6 is pressed, and the compressed air within pump chamber 8 is transferred to the medicine discharge passage (9, 10, 12, 3d, 13) and nozzle passage 14. Consequently, powder medicine 16 within nozzle passage 14 and stirred flow forming chamber 13 is discharged from outlet opening 4d to the nasal cavity. Besides, used nozzle member 4 is detached, and may be thrown out or reused by the recycling.

As shown in FIG. 3, main body 2 and powder medicine receiving container 20 are received in a take-along receiving case or receiving box 30. Receiving case 30 includes a receiving member 31, and a lid member 32 arranged to open and close receiving member 31.
Receiving member 31 includes a recessed portion 33a receiving main body 2 removably, and recessed portions 33b each receiving powder medicine receiving container 20 removably. Powder medicine receiving container 20 is inserted into one recessed portion 33b to expose external thread portion 4c serving as the mounting portion above. Accordingly, it is possible to further readily perform the mounting method (the connection between nozzle member 4 and main body 2 (stirred flow forming member 3)) as described above. Moreover, it is possible to carry out multiple administration because receiving case 30 is provided with a plurality of recessed portions 33b each receiving one powder medicine receiving container 20.

In the device according to the first embodiment, nozzle member 4 is used, instead of the capsule, as powder medicine receiving container 20 for receiving the powder medicine. Accordingly, it is possible to reduce or eliminate the defects produced by using the capsule. In a case in which the sort or the dose of the powder medicine is changed, it is possible to change the sort or the dose of the powder medicine received in nozzle member 4.

In this device according to the first embodiment, sheet-shaped seal members 15a and 15b seal inlet opening 4b and outlet opening 4d of nozzle passage 14 receiving the powder medicine, respectively. Seal members 15a and 15b are detached at the time of the use. In a case in which the hole is formed in the capsule by using the needle, position and shape of hole are varied at every administration (at every boring operation), and this variation of position and shape of hole may cause variation in the discharge state. On the other hand, in the device according to the first embodiment, the position and the shape of the passage are not varied at every administration. Accordingly, it is possible to ensure the stable discharge state of the powder medicine to reduce the variation at every administration.

In this device according to the first embodiment, it is possible to use in a cleaner state by changing nozzle member 4, relative to repeating use of one nozzle member 4.

In the device according to the first embodiment, there is no need to provide constructions for the capsule, such as the needle and a capsule support portion, and accordingly it is possible to simplify construction of the device, and to reduce the number of the components. Therefore, it is possible to reduce the trouble of manufacturing, and to reduce the manufacturing cost.

In this example, carry-along receiving case 30 receives main body 2 and a plurality of powder medicine receiving containers 20 together. Accordingly, it is possible to facilitate the handling of these members, and to grasp frequency of administration readily visually.

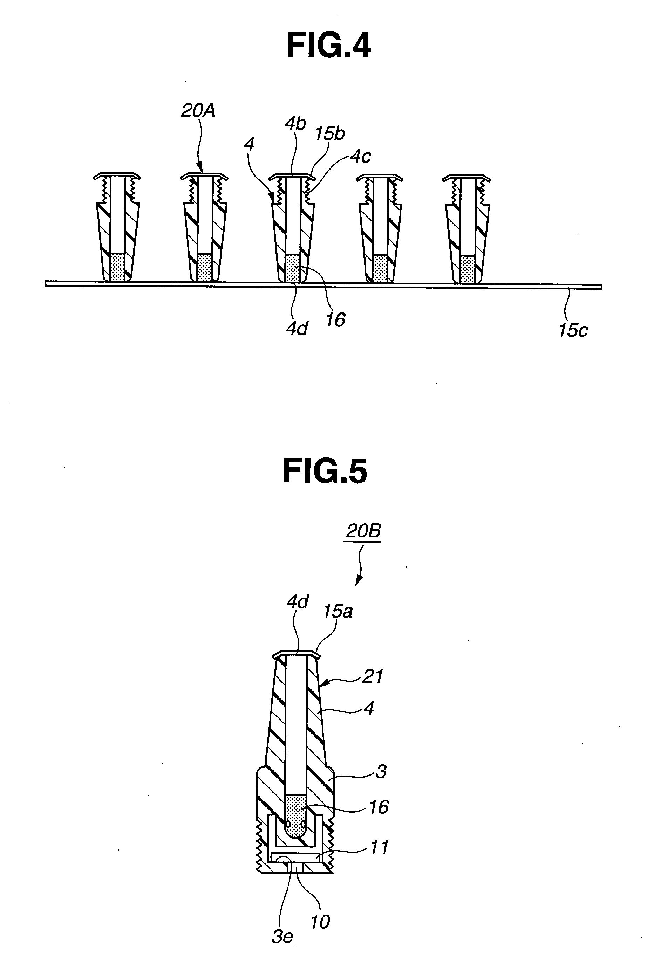

(Second embodiment) FIG. 4 shows a sectional view showing powder medicine receiving containers 20A according to a second embodiment of the present invention. The powder medicine receiving container of FIG. 4 is substantially identical to the structure of FIG. 2 in most aspects as shown by the use of the same reference numerals.

Powder medicine receiving containers 20A commonly use a seal member attached on outlet openings 4d or inlet openings 4b of a plurality of nozzle members 4. Moreover, powder medicine receiving containers 20A may commonly use the other seal member attached on the other openings of outlet openings 4d and inlet openings 4b of nozzle members 4. Accordingly, it is possible to reduce manufacturing cost, and to facilitate the handling of powder medicine receiving containers 20A readily. Besides, each powder medicine receiving container 20A can be employed in powder medicine administering device 1 according to the first embodiment of the present invention. That is, powder medicine receiving container 20A can be attached, for the use, to main body 2 (stirred flow forming member 3) according to the first embodiment.

Accordingly, it is possible to reduce the cost of manufacturing, and to facilitate the handling of powder medicine receiving containers 20A.

(Third embodiment) FIG. 5 shows a sectional view showing a powder medicine receiving container 20B according to a third embodiment of the present invention. The powder medicine receiving container of FIG. 5 is substantially identical to the structure of FIG. 2 in most aspects as shown by the use of the same reference numerals.

In the device according to the first and second embodiments, each of powder medicine receiving containers 20 and 20A uses nozzle member 4 as a main member. On the other hand, in the device according to the third embodiment, nozzle member 4 is integrally formed with stirred flow forming member 3 to form a stirred flow forming nozzle member 21. A powder medicine receiving container 20B uses stirred flow forming nozzle member 21 as a main member. In this case, the main body of the powder medicine administering device is formed by pump member 6, base member 5, and air introduction portion 7. That is, the device according to the third embodiment is different in separation method of a plurality of components, from the devices according to the first and second embodiments. However, in this example, it is also possible to reduce or eliminate the defects caused by using the capsule, like the first and second embodiments. Moreover, it is advantageous to use in a cleaner state by changing stirred flow forming nozzle member 21, relative to repeating use of one stirred flow forming nozzle member.

In this example, stirred flow forming member 3 includes check valve 11 arranged to restrict leakage of powder medicine 16 from air passage 10. Therefore, seal member 15a is provided only on outlet opening 4d. In the device according to the third embodiment, it is possible to reduce number of the components, to reduce the trouble of the manufacturing, and to reduce the manufacturing cost, relative to the devices according to the first and second embodiments. Accordingly, it is further advantageous to omit the trouble at the time of mounting of powder medicine receiving container 20B.

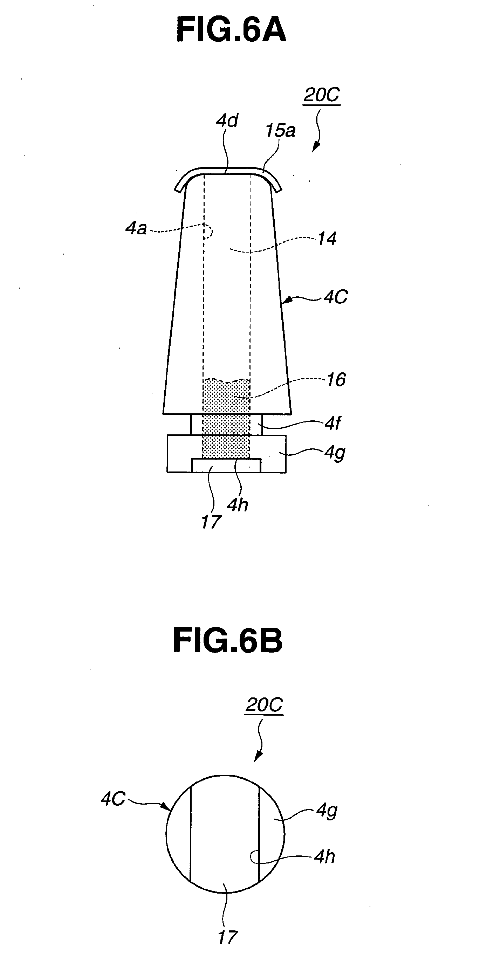

(Forth embodiment) FIG. 6A shows a longitudinal sectional view showing a powder medicine receiving container 20C according to a fourth embodiment of the present invention. FIG. 6B shows a view showing a bottom side of powder medicine receiving container 20C of FIG. 6A.

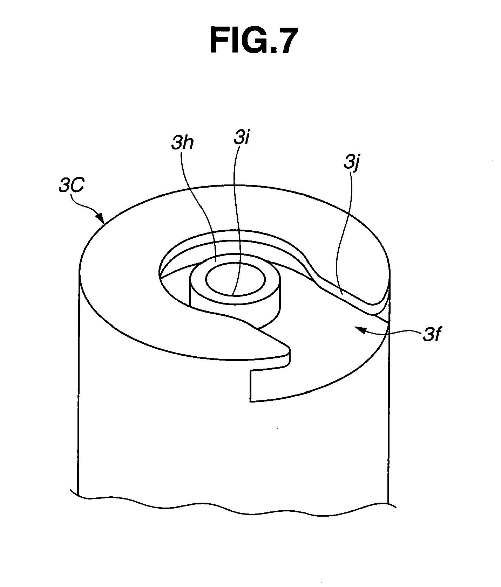

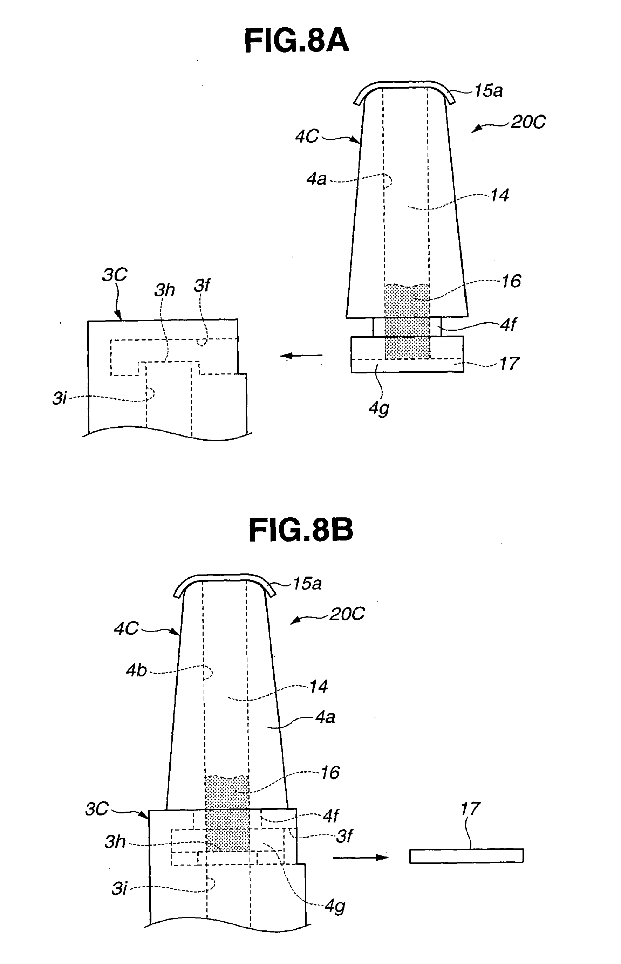

FIG. 7 shows a perspective view showing an upper portion of the main body to which the powder medicine receiving container is attached. FIG. 8A shows a side view showing the mounting operation of powder medicine receiving container 20C to the main body before powder medicine receiving container 20C is attached to the main body. FIG. 8B shows a side view showing powder medicine receiving container 20C and the main body after powder medicine receiving container 20C is attached to the main body. The powder medicine receiving container of FIG. 6A is substantially identical to the structure of FIG. 2 in most aspects as shown by the use of the same reference numerals.

In the device according to the fourth embodiment, stirred flow forming member 3C includes a hollow projection 3h serving as a seal member detaching mechanism or section to detach the seal member when powder medicine receiving container 20C is attached to the main body (stirred flow forming member 3C). Nozzle member 4C includes a bottom portion or radially projecting portion 4g, an annular groove 4f, and a linear groove 4h. Annular groove 4f is formed on a supply side (stirred flow forming member 3's side) of nozzle member 4C, as shown in FIG. 6A. Bottom portion 4g is located at a position upstream of annular groove 4f. Linear groove 4h is formed in bottom portion 4g of nozzle member 4C, and opened in a bottom surface of nozzle member 4C, as shown in FIGS. 6A and 6B. Linear groove 4h has a substantially rectangular section. A band-shaped plate member 17 serving as the seal member is fit in linear groove 4h. In this state, through hole 4a is opened to linear groove 4h.

On the other hand, stirred flow forming member 3C of the main body is formed with a recessed portion 3f and a notch portion 3j in which annular groove 4f and bottom portion 4g of nozzle member 4C are inserted. Recessed portion 3f is formed between a circumferential wall of stirred flow forming member 3C and a circumferential wall of projecting portion 3h. Recessed portion 3f is partially opened in the circumferential wall of stirred flow forming member 3C. Notch portion 3j connects an upper surface of stirred flow forming member 3C and recessed portion 3f. Hollow projection 3h is provided in recessed portion 3f to protrude upward from the lower surface of recessed portion 3f. Hollow projection 3h connects nozzle passage 14 of nozzle member 4C mounted on hollow projection 3h and the medicine discharge passage (through hole 3i) of the main body, to ensure sealing between nozzle passage 14 and the medicine discharge passage. At the mounting operation of nozzle member 4C, hollow projection 3h abuts on linear plate member 17, and extrudes plate member 17 relatively from linear groove 4h provided in the bottom portion 4g of nozzle member 4C.

As shown in FIG. 8A, nozzle member 4C is moved (slid) in a direction perpendicular to the axis (in a direction from right to left in FIG. 8A), bottom portion 4g of nozzle member 4C is inserted to recessed portion 3f of stirred flow forming member 3C, and annular groove 4f of nozzle member 4C is inserted to notch portion 3j of stirred flow forming member 3C. Consequently, as shown in FIG. 8B, nozzle member 4C is mounted to stirred flow forming member 3C. In this mounting operation, plate member 17 serving as the seal member is abutted on hollow projection 3h, relatively pushed by hollow projection 3h, and extruded from linear groove 4h.

In the device according to the fourth embodiment of the present invention, hollow projection 3h is provided as the seal member detaching mechanism. Accordingly, it is possible to detach linear plate member 17 serving as the seal member when power medicine receiving container 20C is mounted to stirred flow forming member 3C of the main body. Hence, it is possible to perform the mounting operation readily, relative to the first, second and third embodiments in which the seal member is detached by the separate operation.

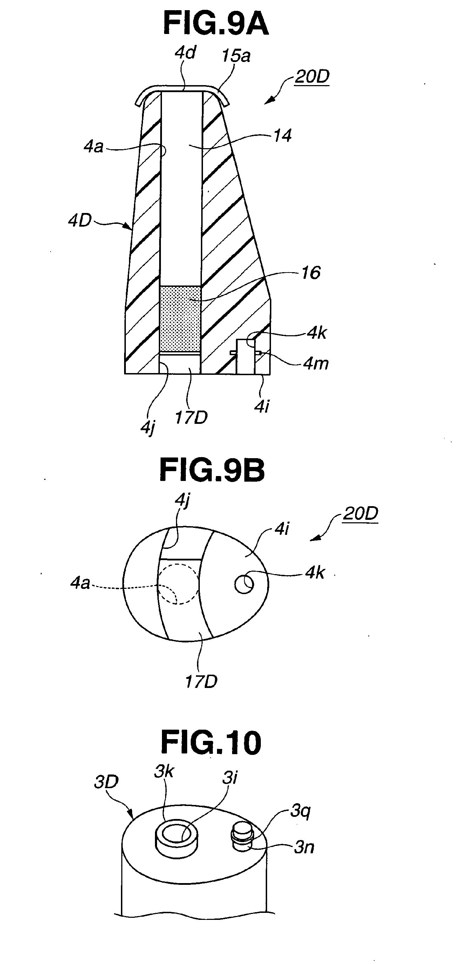

(Fifth embodiment) FIG. 9A shows a longitudinal sectional view showing a powder medicine receiving container according to the fifth embodiment of the present invention. FIG. 9B shows a plan view showing a bottom portion of the powder medicine receiving container of FIG. 9A. The powder medicine receiving container of FIG. 9A is substantially identical to the structure of FIG. 2 in most aspects as shown by the use of the same reference numerals.

In the device according to the fourth embodiment, powder medicine receiving container 20C is moved Linearly, and attached to the main body. On the contrary, in the device according to the fifth embodiment, a powder medicine receiving container 20D is moved rotationally, and attached to the main body.

In the device according to the fifth embodiment, a stirred flow forming member 3D includes a hollow projection 3k serving as the seal member detaching mechanism to detach the seal member when powder medicine receiving container 20D is attached to the main body (stirred flow forming member 3D). Nozzle member 4D includes a bottomed circular hole 4k and an arc groove 4j. Bottomed circular hole 4k is formed in a bottom surface 4i on a supply side (stirred flow forming member 3D's side) of nozzle member 4D. An arc plate member 17D serving as the seal member is fit in arc groove 4j. Arc groove 4j is formed in an arc shape with bottomed hole 4k for its center, and has a substantially rectangular section. Arc plate member 17D is in the form of a plate, and has a substantially rectangular section. In this state, through hole 4a is opened to an upper surface of arc groove 4j.

On the other hand, stirred flow forming member 3D of the main body includes a columnar projecting portion 3n and cylindrical hollow projection 3k. Projecting portion 3n is fit in bottom hole 4k of nozzle member 4D. Hollow projection 3k is loosely inserted to arc groove 4j. Hollow projection 3k connects nozzle passage 14 of nozzle member 4D mounted on hollow projection 3k and the medicine discharge passage (through hole 3i) of the main body, to ensure sealing between nozzle passage 14 and the medicine discharge passage. At the mounting operation of nozzle member 4D, hollow projection 3k abuts on arc plate member 17D, and pushes (extrudes) arc plate member 17D relatively from arc groove 4j provided in the bottom portion of nozzle member 4D. On the other hand, projecting portion 3n is a shaft serving as a center of the rotational movement at the mounting operation of nozzle member 4D. Projecting portion 3n includes an annular protrusion 3q formed on the side wall surface of projecting portion 3n, and arranged to extend radially. Nozzle member 4D includes an annular groove 4m formed in bottom hole 4k. Annular protrusion 3q of projecting portion 3n is engaged with annular groove 4m of bottom hole 4k, and prevents nozzle member 4D from detaching from stirred flow forming member 3D in the axial direction.

In the above mentioned device, projecting portion 3n of stirred flow forming member 3D is inserted into circular bottom hole 4k of nozzle member 4D, and hollow projection 3k of stirred flow forming member 3D is inserted into a portion of arc groove 4j of nozzle member 4D in which arc plate member 17D is not inserted, so that powder medicine receiving container 20D is mounted on stirred flow forming member 3D. Then, powder medicine receiving container 20D is rotationally moved about circular bottom hole 4k along the upper surface of stirred flow forming member 3D. Consequently, arc plate member 17D serving as the seal member is abutted on hollow projection 3k, and extruded from arc groove 4j. Through hole 4a of nozzle member 4D is connected with through hole 3i of stirred flow forming member 3D, to ensure airtightness between through hole 4a and through hole 3i, and accordingly nozzle passage 14 of nozzle member 4D is connected with the medicine discharge passage of the main body. Then, in a state in which outlet opening 4d is directed upward, seal member 15a is detached, so that the device can use.

In the device according to the fifth embodiment, hollow projection 3k is provided as the seal member detaching mechanism. Accordingly, it is possible to detach arc plate member 17D serving as the seal member when power medicine receiving container 20D is mounted to stirred flow forming member 3D serving as the main body. Hence, it is possible to perform the mounting operation readily, relative to the first, second and third embodiments in which the seal member is detached by separate operation.

In the above described embodiments, the main body and the nozzle member are removably connected by the screw connection, and the main body and the stirred flow forming nozzle member are removably connected by the screw connection. Moreover, it is optional to employ another removable connection such as snap fit.

In a case of using the screw connection for connecting two members, the external thread portion and internal thread portion can be interchanged between the two members.

In a case of using the stirred flow forming nozzle member as the powder medicine receiving container, it is possible to form a receiving container for the stirred flow forming nozzle and the main body, like the receiving container according to the first embodiment. Moreover, it is possible to employ the seal member detaching mechanism to detach the seal member.

Moreover, in the stirred flow forming nozzle member, it is optional to generate burble and turbulence of the air flow or the medicine mixed flow by varying the direction of the passage of the stirred flow forming nozzle at right angle or acute angle. That is, it is desirable to generate a stirred state of the medicine and the air, and it is not necessarily require to generate the swirl flow or the vortex flow.

Moreover, it is optional to employ another method or shape such as a cap for covering the end portion formed with the opening, and a plug for closing the opening, as the seal member.

The present invention is also applicable to a powder medicine administering device including a plurality of nozzle members.

In the device according to the embodiments of the present invention, the powder medicine administering device includes the main body formed with the powder medicine discharge passage for discharging the powder medicine, the nozzle member removably attached to the main body, and formed with a nozzle passage connected with the powder medicine discharge passage of the main body, and arranged to receive the powder medicine in the closed state. The nozzle member is arranged to be changed from the closed state to the opened state when the nozzle member is attached to the main body, to discharge the powder medicine received in the nozzle passage. The powder medicine administering device further includes a seal member arranged to be attached to the nozzle member to provide the closed state of the nozzle member to restrict a leakage of the powder medicine received in the nozzle passage. The seal member is detached from the nozzle member to provide the opened state of the nozzle member when the nozzle member is attached to the main body.

In the above-described arrangement, it is possible to reduce or eliminate the defects caused by using the capsule because the nozzle member is used as the container receiving the powder medicine, instead of the capsule. Moreover, it is advantageous to use in a cleaner state by changing the nozzle member, relative to repeating use of one nozzle member.

In the devices according to the embodiments of the present invention, the sheet-shaped seal member closes at least one of the inlet opening and the outlet opening of the nozzle member, and the seal member is detached at the time of the use.

Accordingly, it is possible to certainly readily obtain the sealed state of the portion in which the powder medicine is received. Moreover, it is possible to readily ensure the passages (the medicine discharge passage, the nozzle passage, and so on) with the uniform shape which does not vary at every administration because the sealed state is released by detaching the seal member. It is possible to suppress the variation in the discharge characteristic at every administration (at every boring operation), in comparison with a case in which the hole is formed in the capsule by the needle.

In the devices according to the embodiments of the present invention, the seal member is in the form of sheet.

Accordingly, it is possible to readily attain the sealed state of the powder medicine, and to readily release the sealed state.

In the devices according to the embodiments of the present invention, the main body includes the seal member detaching section arranged to detach the seal member from the nozzle member when the nozzle member is attached to the main body.

Accordingly, it is possible to omit the trouble to detach the seal member, and to readily attain the administration enabling state in which the powder medicine can be administered.

In the devices according to the embodiments of the present invention, the check valve is located on the upstream position of the portion of the nozzle passage in which the powder medicine is received, and arranged to suppress the reverse flow of the powder medicine.

In the above described arrangement, it is possible to omit the seal member because the check valve suppresses the leakage of the powder medicine to the upstream side. Accordingly, it is possible to reduce the number of the components, to omit the trouble of the manufacturing, and to reduce the manufacturing cost. Moreover, it is possible to omit the trouble of detaching the seal member.

In the devices according to the embodiments of the present invention, the stirred flow forming section includes the circumferential side wall surface, and the air passage to introduce the air flow in the tangent direction of the side wall surface.

Accordingly, it is possible to promote the agitation of the air and the powder medicine because the stirred flow flowing along the circumferential side wall surface is formed by the introduced air flow.

This application is based on a prior Japanese Patent Application No. 2005-141496. The entire contents of the Japanese Patent Application No. 2005-141496 with a filing date of May 13, 2005 are hereby incorporated by reference.

Although the invention has been described above by reference to certain embodiments of the invention, the invention is not limited to the embodiments described above. Modifications and variations of the embodiments described above will occur to those skilled in the art in light of the above teachings. The scope of the invention is defined with reference to the following claims.

## Claims

1. A powder medicine administering device comprising:
a main body (2) formed with a powder medicine discharge passage (9, 10) for discharging a powder medicine (16); and
a nozzle member (4; 21) removably attached to the main body (2), and formed with a nozzle passage (14) connected with the powder medicine discharge passage (9, 10) of the main body (2), and arranged to receive the powder medicine (16) in a closed state, the nozzle member being arranged to be changed from the closed state to an opened state when the nozzle member (4; 21) is attached to the main body (2), to discharge the powder medicine received in the nozzle passage (14).

2. The powder medicine administering device as claimed in Claim 1, wherein the powder medicine administering device further includes a seal member (15a; 15b; 15c; 17; 17D) arranged to be attached to the nozzle member to provide the closed state of the nozzle member to restrict a leakage of the powder medicine (16) received in the nozzle passage; and the seal member (15a; 15b; 15c; 17; 17D) is detached from the nozzle member (4; 21) to provide the opened state of the nozzle member when the nozzle member (4) is attached to the main body (2).

3. The powder medicine administering device as claimed in Claim 2, wherein the main body (2) includes a seal member detaching section (3h; 3k) arranged to detach the seal member (17; 17D) from the nozzle member (4) when the nozzle member (4) is attached to the main body (2).

4. The powder medicine administering device as claimed in Claim 2, wherein the nozzle member (4) includes an inlet opening (4b) and an outlet opening (4d); and the seal member (15a; 15b; 15c) is in the form of a sheet, and the seal member (15a; 15b; 15c) closes one of the inlet opening (4b) and the outlet opening (4d) of the nozzle member (4, 21).

5. The powder medicine administering device as claimed in Claim 4, wherein the seal member (15a; 15b; 15c; 17; 17D) is a first seal member (15a; 15b; 15c; 17; 17D) arranged to close one of the inlet opening (4b) and the outlet opening (4d) of the nozzle member (4, 21); and the powder medicine administering device further comprises a second seal member (15a; 15b; 15c; 17; 17D) arranged to close the other of the inlet opening (4b) and the outlet opening (4d) of the nozzle member (4; 21).

6. The powder medicine administering device as claimed in Claim 2, wherein the powder medicine administering device further comprises a plurality of nozzle members (4); and the seal member (15c) closes inlet openings (4b) of the nozzle members (4).

7. The powder medicine administering device as claimed in Claim 2, wherein the powder medicine administering device further comprises a plurality of nozzle members (4); and the seal member (15c) closes outlet openings (4d) of the nozzle members (4).

8. The powder medicine administering device as claimed in Claim 4, wherein the nozzle member (21) includes a stirred flow forming portion (3) located at a position upstream of the nozzle passage (14), and arranged to produce a stirred flow of the air.

9. The powder medicine administering device as claimed in Claim 8, wherein the nozzle member (4, 21) includes a check valve (11) arranged to close the inlet opening (4b) to restrict a leakage of the powder medicine.

10. The powder medicine administering device as claimed in Claim 3, wherein the nozzle member (4) includes a bottom portion (4g) abutted on the main body (2), and a groove (4h, 4j) formed in the bottom portion (4g); and the seal member (17, 17D) is a plate member fit in the groove (4h, 4j) to restrict the leakage of the powder medicine (16).

11. The powder medicine administering device as claimed in Claim 10, wherein the seal member detaching section is a hollow projection (3h, 3k) projecting from the main body (2) toward the nozzle member (4, 21) when the nozzle member (4, 21) is attached to the main body (2); the hollow projection (3h, 3k) of the main body (2) includes a through hole (3i) serving as the powder medicine discharge passage (9, 10); and the nozzle passage (14) of the nozzle member (4C, 4D) is connected with the through hole (3i) of the hollow projection (3h, 3k) of the main body (2).

12. The powder medicine administering device as claimed in Claim 11, wherein the groove (4h) of the nozzle member (4) is a linear groove; and the plate member (17) is a linear plate member (17) to be loosely engaged with the linear groove (4h) of the nozzle member (4).

13. The powder medicine administering device as claimed in Claim 12, wherein the plate member (17) is detached from the nozzle member (4) by a linear movement of the hollow projection (3h) of the main body (2) along the linear groove (4h) of the nozzle member (4) when the nozzle member (4) is attached to the main body (2).

14. The powder medicine administering device as claimed in Claim 11, wherein the groove (4j) of the nozzle member (4) is an arc groove (4j); and the plate member (17D) is an arc plate member (17D) to be loosely engaged with the arc groove (4j) of the nozzle member (4).

15. The powder medicine administering device as claimed in Claim 14, wherein the arc plate member (17D) is detached from the nozzle member (4) by an arc movement of the hollow projection (3k) of the main body (2) along the arc groove (4j) of the nozzle member (4) when the nozzle member (4) is attached to the main body (2).

16. The powder medicine administering device as claimed in Claim 15, wherein the main body (2) includes a projecting portion (3n) extending along the hollow projection (3k); and the projecting portion (3n) is a center shaft serving as a center of the arc groove (4j).

17. A powder medicine administering device comprising:
a main body (2); and
a nozzle member (4, 21) removably attached to the main body (2), and formed with a nozzle passage (14) for discharging a powder medicine (16), the nozzle passage being arranged to receive the powder medicine (16).

18. The powder medicine administering device as claimed in Claim 17, wherein the nozzle member (21) includes a stirred flow forming chamber (13) to stir the powder medicine (16); and the stirred flow forming chamber (13) is located near the main body (2) when the nozzle member (21) is attached to the main body (2).

19. The powder medicine administering device as claimed in Claim 17, wherein the nozzle member (4, 21) includes a circumferential wall portion defining the stirred flow forming chamber (13); and the circumferential wall portion includes an air passage (3d) extending in a tangent direction of the circumferential wall portion.

20. The powder medicine administering device as claimed in Claim 17, wherein the nozzle member (4, 21) includes a check valve (11) arranged to close the inlet opening (4b) to restrict a leakage of the powder medicine.
